(19) **Europäisches Patentamt**

**European Patent Office**

**Office européen des brevets**

(11) **EP 1 024 830 B1**

(12) **FASCICULE DE BREVET EUROPEEN**

(45) Date de publication et mention
de la délivrance du brevet:
**24.11.2004 Bulletin 2004/48**

(51) Int Cl.[7]: **A61K 39/39**, A61K 9/127

(21) Numéro de dépôt: **98944010.2**

(86) Numéro de dépôt international:
**PCT/FR1998/001991**

(22) Date de dépôt: **17.09.1998**

(87) Numéro de publication internationale:
**WO 1999/016468 (08.04.1999 Gazette 1999/14)**

(54) **VECTEURS D'ANTIGENES SOUS FORME DE VESICULES MULTILAMELLAIRES**

ANTIGENVEKTOREN BESTEHEND AUS MULTILAMELLARFÖRMIGEN BLÄSCHEN

ANTIGEN VECTORS IN THE FORM OF MULTILAMELLAR VESICLES

(84) Etats contractants désignés:
**BE DE ES FR GB IT**

(30) Priorité: **29.09.1997 FR 9712085**

(43) Date de publication de la demande:
**09.08.2000 Bulletin 2000/32**

(73) Titulaire: **CAPSULIS**
**33600 Pessac (FR)**

(72) Inventeurs:
- **MAHY, Patrick**
  **F-33400 Talence (FR)**
- **DELORD, Brigitte**
  **F-33600 Pessac (FR)**
- **AMEDEE, Joelle**
  **F-33600 Pessac (FR)**
- **FREUND, Olivier**
  **F-33400 Talence (FR)**
- **ROUX, Didier**
  **F-33700 Merignac (FR)**
- **LAVERSANNE, René**
  **F-33600 Pessac (FR)**
- **GAUBERT, Sophie**
  **F-59000 Lille (FR)**
- **KAISERLIAN, Dominique**
  **F-69007 Lyon (FR)**

(74) Mandataire: **Giraud, Françoise et al**
**Cabinet Beau de Loménie**
**158, rue de l'Université**
**75340 Paris Cedex 07 (FR)**

(56) Documents cités:
**WO-A-93/19735**　　　**WO-A-95/22961**
**WO-A-98/02144**　　　**FR-A- 2 735 658**

- **N.M. WASSEF ET AL.: "LIPOSOMES AS CARRIERS FOR VACCINES" IMMUNOMETHODS, vol. 4, juin 1994, pages 217-222, XP000601673 SAN DIEGO, CA, US**

Remarques:
Le dossier contient des informations techniques présentées postérieurement au dépôt de la demande et ne figurant pas dans le présent fascicule.

## Description

La présente invention concerne des vecteurs d'antigènes sous forme de vésicules multilamellaires et des compositions renfermant des antigènes encapsulés dans ces vésicules.

Plus de 100 ans après la découverte des agents infectieux par Louis Pasteur, la question suivante est toujours d'actualité: comment préparer le système immunitaire à être durablement efficace contre une infection, dans des conditions de sécurité totale ? L'enjeu des recherches est non seulement de mettre au point de nouveaux vaccins, mais aussi d'améliorer ceux déjà existants, en réduisant le nombre d'injections.

De nos jours, la plupart des vaccins viraux sont des virus vivants atténués. Cette stratégie a été moins fructueuse contre les bactéries puisqu'un seul vaccin bactérien atténué est utilisé à ce jour : le BCG. La démarche d'atténuation des microorganismes a fait d'énormes progrès grâce aux techniques de Biologie Moléculaire qui rendent possible de modifier la fonction de virulence de ces agents pathogènes. Néanmoins, il est difficile de concilier l'atténuation de cette virulence et une stimulation efficace des défenses immunitaires. Les vaccins dits moléculaires, à base d'antigènes purifiés exigent le concours d'adjuvants à propos desquels toute une recherche reste encore à développer. A titre d'exemple, le premier vaccin de ce type a été mis au point contre l'hépatite B en 1987 à partir d'une protéine de l'enveloppe virale (antigène HBs) produite par les cellules animales. Ces vaccins moléculaires sont encore peu nombreux car les antigènes purifiés sont rapidement éliminés par l'organisme. De plus, les antigènes purifiés, injectés seuls sont peu efficaces et nécessitent l'emploi de substances qui favorisent leur reconnaissance par le système immunitaire et qui ralentissent leur dégradation : les adjuvants. Actuellement, l'hydroxyde d'aluminium $Al(OH)_3$, découvert en 1926, est un adjuvant autorisé chez l'homme. De nouvelles substances ont été découvertes au cours des dernières années : dérivés synthétiques de la paroi des mycobactéries (muramyldipeptide et ses dérivés).

Cependant, la démonstration de l'innocuité de ces nouveaux agents nécessite une multitude de tests, avant qu'ils puissent être utilisés dans des vaccins à usage humain. Enfin se pose le problème du nombre d'injections ; car en l'absence de rappels, la mémoire immunitaire devient alors insuffisante.

La vaccination moderne, en particulier celle utilisant des protéines recombinantes, est basée sur la réponse immunitaire liée à l'administration d'un antigène par différentes voies (intramusculaire, per os). Cependant, celle-ci peut être mal stimulée, du fait soit de la biodisponibilité insuffisante de l'antigène (trop courte durée de vie, protéolyse et dilution dans les fluides biologiques), soit de l'absence d'une capture et d'une présentation efficace par les cellules présentatrices d'antigènes (accessibilité des cellules dendritiques au site d'immunisation, conformation de l'antigène ne permettant pas à la fois association aux molécules de classe I et de classe II du Complexe Majeur d'Histocompatibilité), soit d'une reconnaissance imparfaite des déterminants antigéniques par les cellules immunitaires, même lorsque la séquence protéique est conçue de façon optimale (ex. : les glycoprotéines associées au virus HIV, ou les marqueurs tumoraux).

Il existe donc un très grand intérêt à vectoriser ces molécules afin de tenter de pallier aux limitations décrites ci-dessus.

Classiquement, la vaccination vise une réponse immunitaire humorale. Ce système protège l'individu vacciné d'une exposition éventuelle à l'agent pathogène, prévenant de la sorte, la dissémination d'une infection extra-cellulaire. Cependant, il est impuissant vis-à-vis d'une infection intracellulaire. La réponse immunitaire cellulaire est donc le complément indispensable à l'élimination de la cellule infectée. Par conséquent une vaccination idéale devrait favoriser les deux types d'immunité, cellulaire et humorale. La réponse immunitaire cellulaire permet l'élimination d'agents pathogènes intracellulaires tels que les virus ou la destruction des cellules cancéreuses. A l'heure actuelle, l'obtention d'une réponse immunitaire adéquate par administration d'un antigène se heurte encore à de nombreuses difficultés. Il est de ce fait encore impossible d'obtenir une vaccination contre les antigènes tumoraux spécifiques (ex: cancer du sein) ou contre des antigènes faiblement immunogènes (ex: glycoprotéines associées au VIH).

Il existe donc un besoin évident d'une méthode qui permettrait de vectoriser les antigènes afin d'induire une réponse cellulaire en excluant d'éventuels chocs anaphylactiques ou une protéolyse dans le sérum.

Une multitude de maladies pourrait bénéficier de l'administration de protéines ou de peptides antigéniques thérapeutiques vectorisés. Ceci permettrait de réduire les effets secondaires indésirables liés à la trop grande quantité d'antigènes à administrer afin de "susciter" la réponse immunitaire (ex: vaccination anticancéreuse).

Il a été proposé dans la littérature d'utiliser des liposomes à la fois comme vecteur d'antigène et comme immunoadjuvant pour l'obtention de vaccins. La demande internationale WO 95/22961 enseigne une sélection particulière des phospholipides entrant dans la composition de tels liposomes, cette sélection étant destinée à améliorer la réponse immunitaire, et cela, que les antigènes soient simplement utilisés en présence des liposomes ou qu'ils soient encapsulés dans ces liposomes.

Les inventeurs de la présente invention ont mis au point, pour différentes applications dans des domaines totalement différents de ceux de la présente invention, des vésicules multilamellaires qui présentent une structure dite en "oignon" et sont constituées, de leur centre jusqu'à leur périphérie, d'une succession de couches lamellaires séparées par un milieu liquide. Ces vésicules peuvent être obtenues par un procédé comprenant la préparation d'une phase lamellaire cristal-liquide et sa transformation par application d'un cisaillement. Un tel procédé est en particulier décrit dans le brevet WO 93/19735 issu du brevet français FR-2 689 418 ou WO 95/18601 introduits ici par référence.

Selon le brevet français FR-2 689 418, cette transformation peut être faite lors d'une étape de cisaillement homogène de la phase cristal-liquide, ce qui conduit à des vésicules encore appelées microcapsules de taille contrôlée. Toutefois, en jouant sur la formulation de la phase lamellaire cristal-liquide, en particulier sur la nature des tensioactifs entrant dans sa composition, la transformation de cette phase cristal-liquide en vésicules peut être obtenue par simple sollicitation mécanique, en particulier lors du mélange des constituants.

Les recherches menées par les inventeurs les ont amenés à découvrir que l'utilisation des technologies décrites ci-dessus permettait la mise au point de nouveaux vecteurs multilamellaires de petite taille, présentant une grande efficacité d'encapsulation et une grande facilité de préparation, et pouvant être utilisés pour vectoriser des antigènes, avec pour résultat d'augmenter la réponse immunitaire de l'organisme contre l'antigène encapsulé.

Un tel vecteur présente à bien des égards les avantages recherchés pour un adjuvant de vaccination.

Un autre avantage est que toutes les molécules entrant dans la préparation des compositions de l'invention sont disponibles commercialement.

D'autres avantages sont liés au procédé de préparation particulièrement simple des vésicules multilamellaires utiles selon l'invention, qui permet d'utiliser une grande variété de tensioactifs.

Un autre avantage, également lié au procédé de préparation des vésicules multilamellaires utiles selon l'invention, est qu'il permet, comme cela ressort de l'exposé qui va suivre, de coencapsuler plusieurs antigènes dans la même vésicule, permettant ainsi l'accès éventuellement à des vaccins plurivalents ou de coencapsuler avec un ou plusieurs antigènes des additifs permettant de fonctionnaliser les vésicules, permettant par exemple, d'améliorer le ciblage de la composition ou d'influer sur l'orientation (humorale ou cellulaire) de la réponse immunitaire.

Un autre avantage, lié lui aussi essentiellement au procédé utilisé pour préparer les vésicules à structure en oignon utilisées selon l'invention, réside dans le fait que l'on incorpore les actifs et les additifs préalablement à la formation des vésicules, ce qui permet un excellent rendement d'encapsulation, d'où une meilleure efficacité et une économie très importante pour des molécules extrêmement coûteuses.

De plus, un autre avantage lui aussi lié au procédé utilisé pour la préparation des vésicules utilisées selon l'invention est que l'on peut indifféremment incorporer des actifs ou des additifs soit hydrosolubles, soit liposolubles.

Plus précisément, la présente invention propose un nouveau moyen de vectorisation des antigènes, en vue d'augmenter la réponse immunitaire de l'organisme contre cet ou ces antigènes. La présente invention vise plus précisément une méthode pour administrer un antigène permettant d'améliorer significativement la réponse immunitaire aussi bien cellulaire qu'humorale.

Ainsi, selon une de ses caractéristiques essentielles, l'invention concerne un nouveau vecteur d'antigène, constitué d'une dispersion de vésicules multilamellaires au sein desquelles ledit antigène se trouve au moins partiellement encapsulé, lesdites vésicules étant constituées de bicouches comprenant au moins un agent tensioactif, lesdites bicouches étant concentriques et conférant auxdites vésicules une structure en oignon.

Par antigène, on entend toute substance qui induit une réponse du système immunitaire et/ou la production d'anticorps. Une substance antigénique comprend au moins un déterminant antigénique ou épitope, composé de motifs moléculaires reconnus par des anticorps spécifiques et/ou les cellules du système immunitaire.

Les antigènes utilisés selon l'invention sont avantageusement des antigènes de type protéique. Il pourra, en particulier, s'agir d'antigènes constitués de protéines naturelles ou recombinantes, ou de peptides.

On pourra également utiliser les vésicules de l'invention comme vecteurs d'antigènes de type glycoprotéines ou polysaccharides.

Les vésicules de l'invention pourront contenir un antigène ou un mélange d'antigènes. A titre de mélange d'antigènes, on citera en particulier les mélanges obtenus soit par synthèse ou par biotechnologie, ou par extraction à partir des microorganismes concernés.

Par structure en "oignon", on entend, comme exposé précédemment, une structure multilamellaire, dans laquelle les vésicules de forme sensiblement sphérique sont constituées d'une succession de bicouches concentriques et, cela, du centre à la périphérie des vésicules, d'où le nom de structure en oignon utilisé, par analogie, pour qualifier de telles structures.

De telles structures sont avantageusement obtenues par incorporation d'au moins un antigène dans une phase lamellaire cristal-liquide comprenant au moins un agent tensioactif puis transformation de cette phase cristal-liquide lamellaire en une phase dense de vésicules multilamellaires de petite taille.

Ainsi, selon une autre caractéristique essentielle de l'invention, elle concerne un procédé de préparation d'une composition contenant au moins un antigène, selon lequel on prépare une phase cristal-liquide lamellaire incorporant ledit antigène et on provoque le réarrangement de ladite phase cristal-liquide en vésicules multilamellaires par application d'un cisaillement.

Ce cisaillement pourra être un cisaillement homogène, ce qui présente l'avantage de conduire à des vésicules de taille parfaitement homogène. Toutefois, une simple agitation mécanique pourra s'avérer suffisante pour conduire à la formation des vésicules multilamellaires de l'invention.

Selon une autre caractéristique encore, l'invention concerne les produits susceptibles d'être obtenus par ce procédé.

Selon le brevet français FR-2 689 418, cette transformation peut être faite lors d'une étape de cisaillement homogène de la phase cristal-liquide, ce qui conduit à des vésicules ou microcapsules de taille contrôlée. Toutefois, en jouant sur la formulation de la phase lamellaire cristal-liquide, en particulier sur la nature des tensioactifs entrant dans sa composition, la transformation de cette phase cristal- liquide en vésicules peut être obtenue par simple sollicitation mécanique, en particulier lors du mélange des constituants.

La formulation fait avantageusement intervenir un mélange de molécules tensioactives. Il est généralement utilisé au moins deux tensioactifs différents ayant des balances hydrophile-lipophile différentes, ce qui permet de régler en continu les propriétés des bicouches et ainsi de contrôler l'apparition de l'instabilité qui gouverne la formation des vésicules multilamellaires.

Il semble que cette structure soit responsable des résultats particulièrement avantageux obtenus, et que les vésicules multilamellaires de l'invention permettent à l'antigène d'arriver intact jusqu'aux cellules présentatrices de l'antigène (CPA) et de favoriser sa capture par ces cellules. Il semble donc bien que la fonction des vésicules de l'invention est de vectoriser, de protéger et d'améliorer la capture de l'antigène par le système immunitaire.

Selon une variante avantageuse, les vésicules constituant les compositions de la présente invention ont des diamètres inférieurs à 20 μm, de préférence inférieurs à 10 μm, de préférence encore inférieurs à 1 μm, de préférence encore compris entre 0,1 et 1 μm.

Selon une variante avantageuse, les membranes des vésicules contenues dans les compositions de l'invention contiennent au moins un agent tensioactif choisi dans le groupe constitué :

- des phospholipides hydrogénés ou non hydrogénés,
- des acides gras en $C_6$ à $C_{18}$, saturés ou mono- ou polyinsaturés, linéaires ou ramifiés, sous forme d'acide ou de sel d'un métal alcalin, alcalino-terreux ou d'une amine,
- des esters, éthoxylés ou non, de ces mêmes acides gras et

  . de saccharose,
  . de sorbitan
  . de mannitol,
  . de glycérol ou de polyglycérol,
  . de glycol,

- des mono-, di- ou triglycérides ou des mélanges de glycérides de ces mêmes acides gras,
- des alcools gras en $C_6$ à $C_{18}$, saturés ou mono- ou polyinsaturés, linéaires ou ramifiés, éthoxylés ou non,
- des éthers, éthoxylés ou non, de ces mêmes alcools gras et

  . de saccharose,
  . de sorbitan,
  . de mannitol,
  . de glycérol ou de polyglycérol,
  . de glycol,

- des huiles végétales polyéthoxylées, hydrogénées ou non hydrogénées,
- des polymères séquencés de polyoxyéthylène et de polyoxypropylène (poloxamères),
- de l'hydroxystéarate de polyéthylèneglycol,
- des alcools à squelette stérol tel que le cholestérol, le sitostérol.

Les tensioactifs choisis, en particulier les tensioactifs cités ci-dessus, sont avantageusement choisis dans la catégorie des tensioactifs permis par la législation pour l'utilisation pharmaceutique en fonction de la voie d'administration.

On choisira avantageusement parmi les tensioactifs ci-dessus, deux tensioactifs présentant des propriétés relativement différentes, en particulier une balance hydrophile-lipophile (HLB) différente. Le premier tensioactif présentera avantageusement une balance hydrophile-lipophile comprise entre 1 et 6, de préférence entre 1 et 4, alors que le deuxième tensioactif aura une balance hydrophile-lipophile comprise entre 3 et 15, de préférence comprise entre 5 et 15.

Comme on l'a vu précédemment, un meilleur contrôle de la taille des vésicules multicouches pourra être obtenu en procédant selon le brevet FR 2 689 418.

La préparation obtenue après transformation de la phase lamellaire cristal-liquide en vésicules multilamellaires peut ensuite être diluée, en particulier avec un solvant aqueux tel que, par exemple, une solution tampon, une solution saline ou une solution physiologique, pour obtenir ainsi une suspension aqueuse de vésicules.

La technique d'encapsulation utilisée selon la présente invention permet d'atteindre aisément des rendements d'encapsulation très élevés, voire voisins de 100 %. Toutefois, de tels rendements ne sont pas toujours indispensables en fonction des applications visées.

Ainsi, le rendement d'encapsulation du (ou des) antigène(s) dans les compositions de l'invention est avantageusement supérieur à 50 %, de préférence supérieur à 80 %.

Un autre avantage lié à la technique d'encapsulation de l'invention est qu'elle permet de coencapsuler plusieurs antigènes au sein des mêmes vésicules, mais également un ou plusieurs antigènes avec des additifs permettant de fonctionnaliser ces vésicules ; ceci permet en particulier d'améliorer le ciblage. On pourra par exemple coencapsuler des cytokines, qui vont permettre d'influer sur l'orientation (humorale ou cellulaire) de la réponse immunitaire.

L'invention fournit un moyen de vectorisation d'antigènes, en particulier d'antigènes de type protéique, cette vectorisation pouvant être réalisée in vivo après injection par voie intraveineuse ou intrapéritonéale.

Selon une autre de ses caractéristiques essentielles, l'invention concerne une composition contenant au moins un antigène contre lequel on souhaite produire des anticorps spécifiques ou une réponse cellulaire spécifique, dans laquelle ledit (lesdits) antigène(s) se trouve(nt) au moins partiellement encapsulé(s) dans les vésicules multilamellaires d'un vecteur tel qu'il a été défini précédemment.

Les compositions contenant au moins un antigène encapsulé dans les vésicules multilamellaires précédemment décrites peuvent être aussi bien des compositions pharmaceutiques destinées à être utilisées comme vaccins, que des compositions destinées à la production d'anticorps monoclonaux.

Enfin, selon une autre de ses caractéristiques essentielles, l'invention concerne un procédé de préparation des compositions définies précédemment.

Ce procédé comprend les étapes suivantes de :

- préparation d'une phase lamellaire cristal-liquide comprenant au moins un agent tensioactif et incorporant le(les) antigène(s),
- transformation de la phase cristal-liquide en vésicules multilamellaires à structure en oignon, par cisaillement,
- dispersion dans une solution aqueuse pour obtenir la concentration voulue en antigène(s).

Plus précisément, selon ce procédé, on prépare dans une première étape une phase cristal-liquide constituée d'un mélange de molécules amphiphiles et d'une solution aqueuse contenant le (ou les) antigène(s). La phase aqueuse peut contenir, en particulier, un tampon PBS ou tout autre solution physiologique. Bien entendu, le mode opératoire sera adapté pour tenir compte du manque éventuel de solubilité de certains antigènes. On incorporera alors avantageusement dans la phase aqueuse différents additifs ou cosolvants susceptibles de favoriser la solution de l'antigène, tels que le glycérol, le polyéthylèneglycol, le propylèneglycol ou l'urée. L'homme du métier choisira la nature des tensioactifs et leurs proportions de façon à avoir une transformation ultérieure aisée de la phase cristal-liquide en vésicules multilamellaires à structure en oignon. Il pourra choisir, sans que cela soit une obligation, pour ces tensioactifs au moins une molécule amphiphile plutôt hydrophobe présentant de préférence une HLB inférieure à 4 et une autre molécule amphiphile plutôt hydrophile présentant une HLB de préférence supérieure à 5. Un tel choix permettra une transformation aisée de la phase cristal-liquide.

Dans une seconde étape, le mélange homogène constitué de la phase cristal-liquide formée est cisaillé selon un des procédés décrits précédemment. On appliquera avantageusement un cisaillement homogène pour obtenir une taille régulière des vésicules. Toutefois, l'utilisation d'un tel cisaillement homogène ne s'avère pas toujours nécessaire et, à condition de jouer sur la formulation de la phase cristal-liquide, un simple mélange dans un mélangeur industriel peut permettre de transformer la phase lamellaire cristal-liquide en vésicules multilamellaires selon l'invention.

Dans une troisième étape, le mélange constitué des vésicules multilamellaires selon l'invention est dispersé dans un excès de solution aqueuse, par exemple de sérum physiologique, pour amener ce mélange à la concentration en antigène voulue.

L'invention est illustrée par les exemples qui suivent qui montrent qu'il est possible d'envisager l'utilisation des microvésicules multilamellaires de tensioactifs pour la vaccination humaine et animale avec les avantages suivants :

1) Il est possible de vectoriser un ou plusieurs antigènes, séparément ou ensemble dans des vésicules multilamellaires de petite taille.
2) On peut envisager, compte tenu de l'augmentation de la réponse immunitaire, l'utilisation d'une quantité moindre d'antigène. Ceci peut être important lorsqu'on envisage de recourir à des antigènes recombinants et/ou coûteux.
3) L'amplification de la réponse immune par la microencapsulation permet d'envisager l'utilisation de molécules faiblement immunogènes qui, seules, ne conduisent pas à une réponse immunitaire suffisante.

4) La rapidité du développement de la réponse immunitaire permet d'envisager une diminution des écarts entre la mise en oeuvre des rappels successifs. En effet, au dernier rappel, seules 30% des personnes traitées se présentent et donc 70% des individus, bien qu'ayant subi une première injection, ne sont pas immunisés efficacement. Un délai plus bref entre les rappels devrait assurer une meilleure vaccination de la population.

5) L'augmentation de la réponse immune cellulaire est la garantie d'une meilleure vaccination.

6) Le fait que l'effet d'amplification de la réponse immunitaire d'un antigène encapsulé soit encore plus net avec la présence d'un adjuvant tel que le QS21 permet d'envisager (sans obligation) des couplages technologiques.

En conclusion, les résultats démontrent un effet amplificateur de la réponse immunitaire qui est très général et peut potentiellement être appliqué dans de nombreux vaccins humains et animaux ou pour la production de compositions contenant des antigènes.

Les exemples qui suivent sont donnés à titre purement illustratif et non limitatif de l'invention.

L'exemple 3 est illustré par les figures 1 à 4 qui donnent respectivement :

- figure 1 : les résultats concernant la production d'IgG sériques anti-HSA après immunisation selon le protocole IP/ IV défini dans l'exemple 3, à J=19, J=35 et J=50 pour quatre types de traitements réalisés avec HSA encapsulée (1), HSA libre dans PBS (2), HSA libre en mélange avec des vésicules vides (3), et des vésicules vides (4) ;
- figure 2 : la réponse isotypique anti-HSA après immunisation selon le protocole IP/IV défini dans l'exemple 3, exprimée par les titres moyens en isotypes IgG après les quatre types de traitements définis ci-dessous (1, 2, 3 et 4),
- figure 3 : les résultats concernant la production d'IgG sériques anti-HSA après immunisation selon le protocole SC défini dans l'exemple 3 à J=19, J=35, J=50 pour trois types de traitements notés respectivement 1 (avec HSA encapsulée), 2 (avec HSA libre dans PBS) et 3 (avec des vésicules vides),
- figure 4 : la réponse isotypique anti-HSA après trois immunisations selon le protocole SC défini dans l'exemple 3 dans le cas de trois traitements notés 1, 2 et 3 avec HSA encapsulée (1), HSA libre dans PBS (2), des vésicules vides (3),

Les figures 5 et 6 sont données en référence à l'exemple 4 et donnent respectivement les résultats de dosage des cellules lymphocytes B productrices d'anticorps anti-NP d'isotypes IgG par rapport aux cellules spléniques totales après application des deux protocoles d'immunisation définis dans l'exemple 4, comprenant respectivement une immunisation IP (figure 5) et trois immunisations IP (figure 6).

EXEMPLES

Exemple 1

Préparation et caractérisation d'une composition contenant des antigènes encapsulés

On prépare une composition contenant des vésicules multilamellaires et présentant la composition suivante dans laquelle les proportions sont exprimées en pourcentages en poids :

| | |
|---|---|
| phosphatidylcholine (PC90 de Natterman) | 45,5 |
| oléate de potassium | 5 |
| cholestérol | 5 |
| sulfate de cholestérol | 2,5 |
| laureth 4 (lauropal 4 de Witco) | 2 |
| solution aqueuse à 10 mg/ml d'albumine sérique humaine (HSA) | 40 |

en procédant comme exposé ci-dessous.

L'oléate de potassium, le cholestérol, le sulfate de cholestérol et le laureth 4 sont préalablement mélangés sous agitation magnétique à 55°C pendant 1 h. A la pâte obtenue, refroidie à température ambiante sont lentement additionnés sous agitation douce la solution aqueuse d'HSA (ou de l'eau pure pour les microvésicules vides) puis la phosphatidylcholine. Le mélange est ensuite introduit dans une cellule de cisaillement constituée de deux cylindres coaxiaux, dont l'un est fixe et l'autre en rotation. Ce type de dispositif est décrit dans le brevet WO93/19735. Le cisaillement d'environ 10 s$^{-1}$ est maintenu pendant 1 h. L'échantillon est ensuite dispersé dans l'eau stérile, à raison de 250 mg de pâte pour 1 ml d'eau.

Une seconde dilution est effectuée avant l'injection, à raison de 0,5 ml de la solution précédente dans 2 ml d'eau stérile. Pour les échantillons contenant un adjuvant, celui-ci est préalablement incorporé dans l'eau de dispersion à raison de 5 µl de solution aqueuse d'adjuvant (à 20 µg/µl) dans 1,995 ml d'eau. On obtient donc dans la solution injectée une concentration de 20 µg d'antigène pour 100 µl de volume injectable, et dans le cas où l'adjuvant est utilisé sa concentration finale est de 4 µg pour 100 µl.

Le taux d'encapsulation est estimé par dosage après séparation des vésicules du surnageant puis libération de l'antigène. La dispersion de vésicules est ultracentrifugée (40 000 rpm, 1 h). Le culot de vésicules, est dilué 10 fois par une solution à 10 % en sel de déoxycholate de sodium. La suspension de vésicules détruites par le sel de déoxycholate est soumise à un effet vortex intense puis ultracentrifugée (50 000 rpm, 60 minutes). Le dosage de l'antigène est réalisé par spectrophotométrie sur le culot par la technique de Bradford (Analytical Biochemistry, 1976, 72, p. 248) en recourant au dosage "Bio-Rad protein assay" conformément au protocole fourni (Bio-Rad Laboratories, USA).

Les valeurs associées au culot et correspondant à la quantité d'antigène encapsulé (HSA) sont de 80 % $\pm$ 5%.

La taille moyenne des vésicules multilamellaires contenant l'HSA est mesurée par la technique de diffusion dynamique de la lumière. On trouve une taille de 0.218 µm avec une polydispersité de 20 %, ce qui montre qu'on a un échantillon homogène quant à la taille des vésicules.

Exemple 2

Dosage de la réponse immunitaire

a) Protocole de vaccination

On détermine la réponse immunitaire d'un antigène microencapsulé administré in-vivo en recourant à un modèle murin.

Un protocole de vaccination accéléré a été mis en place afin de tester la capacité des vecteurs à induire rapidement une réponse immunitaire. Trois injections sur vingt jours ont été réalisées puis des dosages des immunoglobulines totales (IgG, IgM) à deux et quatre semaines ont été effectués.

Quatre formulations différentes (notées de 1 à 4) contenant les mêmes quantités d'antigènes, et deux formulations témoins (notées 5 et 6) ont été testées :

1) l'antigène seul
2) l'antigène associé à un adjuvant connu (QS21 de AQUILA, USA)
3) l'antigène encapsulé dans les microvésicules multilamellaires de tensioactifs selon l'exemple 1
4) l'antigène encapsulé dans les microvésicules multilamellaires de tensioactifs selon l'exemple 1 associées au QS21
5) les microvésicules multilamellaires de tensioactifs analogues à celles obtenues selon l'exemple 1 mais vides
6) les microvésicules multilamellaires de tensioactifs analogues à celles obtenues selon l'exemple 1 mais vides associées à l'adjuvant QS21.

Toutes les formulations ont été préparées de manière à ce qu'un volume d'injection de 100 µl contienne 20 µg d'antigène (libre ou encapsulé), et lorsqu'il est présent, 4µg d'adjuvant QS21 (*cf.* ci-dessus).

Le modèle murin est celui de souris BALB/c femelles âgées de 8 semaines, d'un poids moyen de 20 g. Dix jours s'écoulent entre la première et la seconde injection intrapéritonéale. La troisième et dernière administration est intraveineuse 10 jours plus tard. Le protocole s'échelonne donc sur 20 jours au total. Le même protocole est suivi pour les six formulations correspondant aux six groupes de quatre souris numérotés de 1 à 6. Chaque groupe a été traité avec la formulation de même numéro.

Les animaux sont maintenus dans des conditions "normales" , nourris ad libitum et normalement hydratés.

Deux prélèvements sanguins (prélèvement rétro-orbitaire) sont effectués à 2 et 4 semaines après la dernière injection, afin de doser les immunoglobulines totales (IgG, IgM) sur le sérum après centrifugation du sang total hépariné.

b) Résultat : dosage de la réponse anticorps totale

Le dosage de la réponse anticorps totale se fait en titrant les immunoglobulines par un test ELISA (Saunders G.C. "Immunoassays in the clinical laboratory" Ed. By Nakamura et al, 1979) à l'aide d'immunoglobulines de lapin anti-immunoglobulines de souris (Institut Pasteur, Paris), marquées à la peroxydase. Les immunoglobulines fixées sont révélées par l'addition du substrat de l'enzyme.

La mesure effectuée dans ce test est classiquement une mesure d'absorbance à 405 nm, le produit de la réaction enzymatique formé dans le test ELISA absorbant la lumière à cette longueur d'onde. La méthode d'analyse utilisée donne des résultats identiques à la mesure de l'absorbance à mi-hauteur de la saturation, mais avec plus de précision pour les faibles valeurs et procède par une méthode permettant d'accéder à une grandeur proportionnelle à la quantité d'antigènes présente dans le sérum par paramétrage des courbes donnant l'absorbance en fonction de la dilution. En effet, la loi de dilution peut être modélisée de la façon suivante :

$$I(D) = I_o + \frac{I_s}{(1 + C_o D)}$$

où :

I(D) est l'absorbance mesurée à la dilution D
$I_o$ est le bruit de fond de la mesure
$I_s$ est la mesure à la saturation (plateau)
$C_o$ est proportionnel à la concentration en anticorps cherchée ($C_o$ a la grandeur d'une concentration).

$I_o$ est mesuré directement. $I_s$ est obtenu à partir d'une expérience dont la réponse est suffisante pour atteindre la saturation. La valeur obtenue est alors utilisée dans le paramétrage des autres mesures de la même série d'expériences, même si pour ces dernières la saturation n'est jamais atteinte.

Le paramétrage des courbes permet de déduire $C_o$ pour chaque expérience, donc un nombre proportionnel à la quantité d'anticorps présents dans le sérum étudié.

Cette méthode est plus fine que la simple mesure de la dilution pour obtenir le demi-plateau, puisqu'elle permet d'obtenir une mesure, même en l'absence de saturation. Elle est donc particulièrement adaptée aux faibles réponses.

Les résultats à deux semaines et à quatre semaines (après la fin du protocole vaccinal) pour l'ensemble des préparations sont donnés dans le tableau 1 suivant.

Les valeurs obtenues par cette méthode sont indiquées dans le tableau 1 ci-dessous et sont donc proportionnelles au taux d'immunoglobulines présentes dans le sang.

TABLEAU 1

| Groupe de souris et N° de formulation | 1 | 2 | 3 | 4 | 5 | 6 |
|---|---|---|---|---|---|---|
| Réponse à 2 semaines | - | - | 145 | 335 | - | - |
| Réponse à 4 semaines | - | 7 | 467 | 894 | - | - |

Le signe "-" correspond à une réponse inférieure à 2, jugée non significative.

On voit donc que, dès 2 semaines, on obtient une réponse immunitaire significative pour les formulations encapsulées (avec ou sans adjuvant). Au bout de quatre semaines, les formules encapsulées donnent une réponse très importante, plus de 50 fois supérieure à la réponse de l'antigène associé à l'adjuvant et plus de 100 fois supérieure lorsque l'on ajoute l'adjuvant QS21 à l'antigène encapsulé.

Exemple 3 : Influence de la voie d'immunisation

D'autres essais d'immunisation par l'HSA sur souris BALB/c ont été menés selon 2 protocoles d'immunisation ci-après dénommés IP/IV et SC comprenant les traitements suivants :

- protocole IP/IV : 2 injections intrapéritonéales (IP) suivies d'une injection intraveineuse (IV),
- protocole SC : 3 injections sous-cutanées (SC).

Les formulations des vésicules étaient identiques à celles de l'exemple 1. Dans tous les cas, la dose d'HSA était de 20 µg par injection de 100 µl. Toutes les injections étaient espacées de 10 jours, soit une injection à J=0, J=10 et J=20. Les prélèvements (rétro-orbitaux) ont eu lieu à J=19, soit un jour avant la dernière injection, et à J=35 et J=50.

Des groupes de 4 souris chacun ont été soumis aux traitements suivants :

- Pour le protocole IP/IV :

  ✓ HSA seule dissoute dans le tampon PBS (témoin)

✓ HSA encapsulée dans les vésicules selon l'invention, à raison de 20μg de protéine dans 5 mg de vésicules dispersées dans le tampon PBS à un volume total de 100 μl

✓ HSA dissoute dans le tampon PBS, additionnée de vésicules vides (20 μg de protéine et 5 mg de vésicules vides dispersées à 100 μl dans le tampon PBS)

✓ Vésicules vides (5 mg de vésicules dispersées à 100 μl dans le tampon PBS)

- Pour le protocole SC :

✓ HSA seule dissoute dans le tampon PBS (témoin)

✓ HSA encapsulée dans les vésicules selon l'invention, à raison de 20μg de protéine dans 5 mg de vésicules dispersées dans le tampon PBS à un volume total de 100 μl

✓ Vésicules vides (5 mg de vésicules dispersées à 100 μl dans le tampon PBS)

Les dosages des anticorps ont été réalisés par ELISA sur les immunoglobulines totales (IgGH+L) à J=19, J=35 et J=50 et sur les isotypes $IgG_1$, $IgG_{2a}$ et $IgG_{2b}$ et $IgG_3$ à J=50.

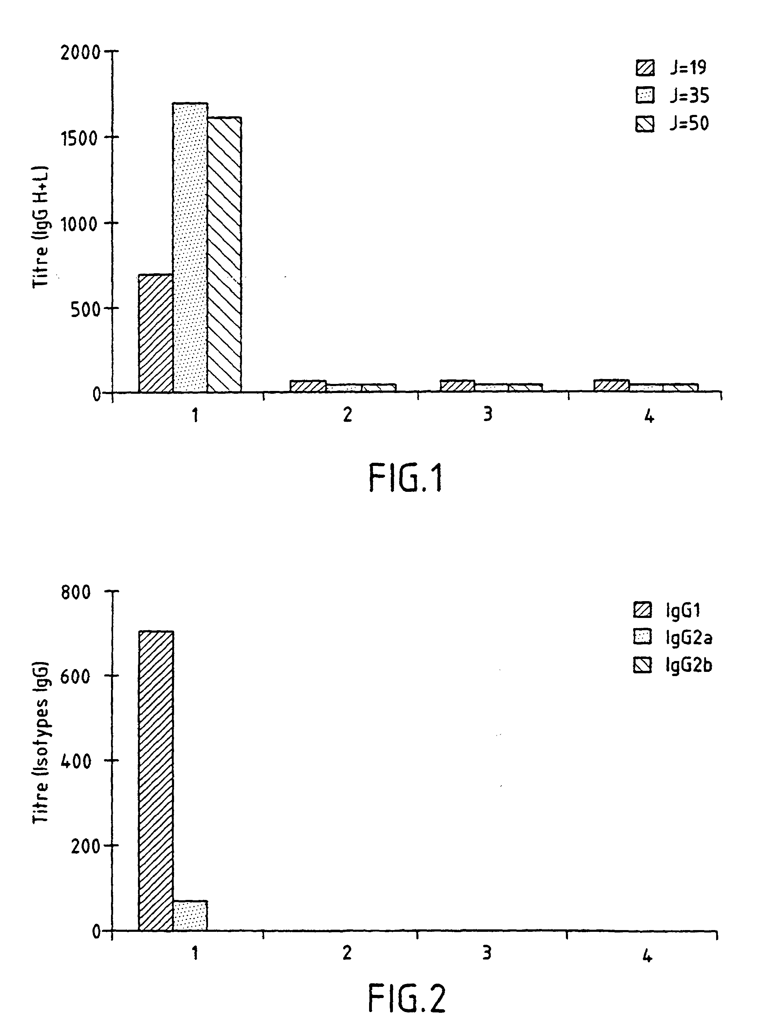

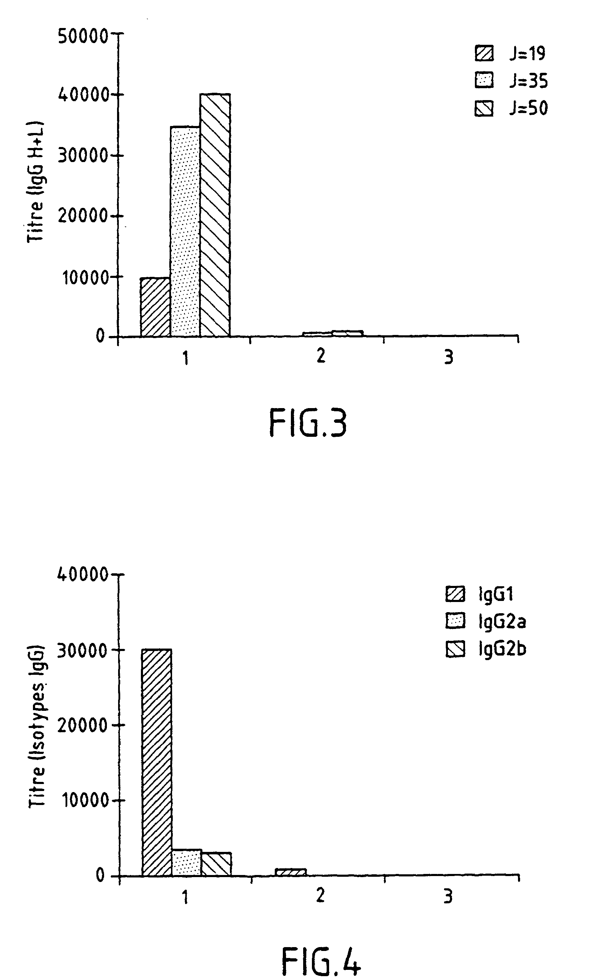

Les résultats sont donnés sur les figures 1 à 4 sur lesquelles apparaissent les titres moyens (moyenne des dosages des sérums individuels). On observe :

1) Une réponse anticorps nulle lorsque l'HSA libre non encapsulée est utilisée, ainsi qu'avec les vésicules vides.

2) Une production importante d'immunoglobulines totales, dans les 2 voies d'administration, lorsque l'HSA est encapsulée, avec un maximum à J = 35

3) Une forte amplification de la réponse IgG totale (H+L) avec l'HSA encapsulée administrée par voie sous-cutanée. Les résultats figurent dans le tableau 2 ci-dessous.

TABLEAU 2

|  | J= 19 | J=35 | J=50 |
|---|---|---|---|
| Protocole IP/IV | 700 | 1700 | 1600 |
| Protocole SC | 10000 | 35000 | 40000 |

4) Pas d'effet adjuvant des vésicules vides puisque l'HSA libre en présence des vésicules vides n'entraîne qu'une très faible réponse.

5) Une production dominante d'IgG1, la présence d'IgG2a dans les 2 protocoles avec des titres beaucoup plus importants dans le cas d'administrations sous-cutanées ainsi que la présence d'IgG2b. Aucune production d'IgG3 n'a pu être observée quelque soit le protocole d'administration.

Cet exemple complète les résultats de l'exemple 2 et démontre que l'encapsulation d'un antigène dans les vésicules multilamellaires selon l'invention amplifie la réponse immunitaire pour différents types d'injection. De plus, ils indiquent que l'effet des vésicules n'est pas un effet adjuvant, puisque l'addition des vésicules vides à l'antigène non encapsulé n'apporte aucune amplification de réponse.

Exemple 4 : Essais avec la protéine NP de la rougeole

Les essais d'immunisation par la protéine NP de la rougeole (protéine interne du virus de la rougeole) ont été menés avec 2 protocoles d'immunisation par injection intrapéritonéale (IP) :

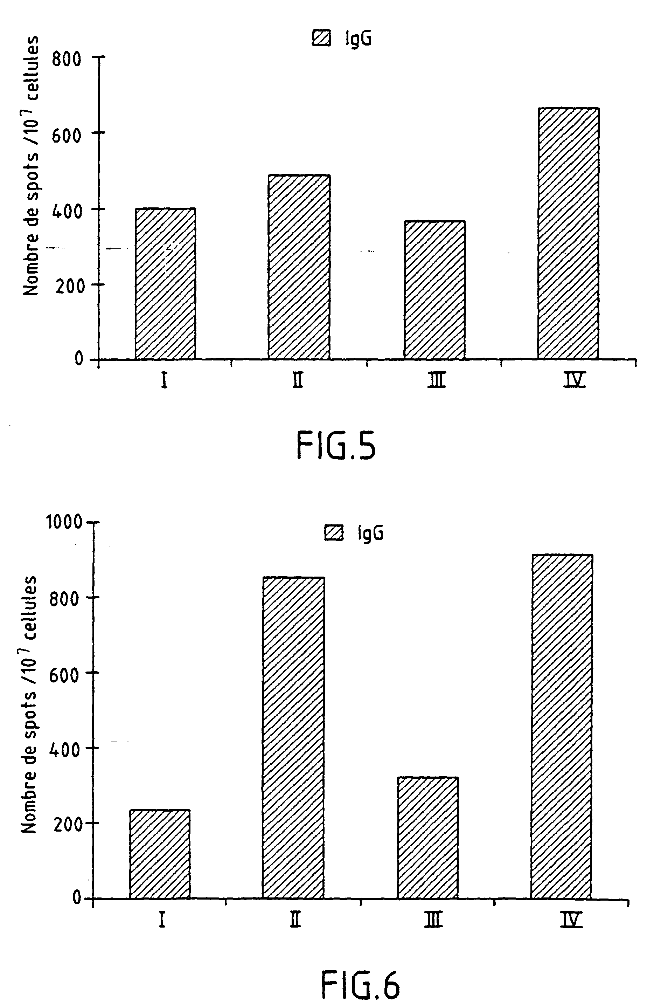

✓ une seule injection (les résultats sont représentés sur la figure 5),

✓ trois injections à 7 jours d'intervalle (les résultats sont donnés sur la figure 6), chacun avec une dose de protéine NP de 30μg, sur des souris BALB/c femelles."

Les vésicules ont été préparées selon la formulation et le mode opératoire de l'exemple 1, avec une dose de 30μg de protéine dans 75 mg de vésicules dispersées dans le tampon PBS (injections de 300 μl titrées à 30μg de protéine).

Dans le cas des injections contenant des vésicules vides, celles-ci étaient dosées à 75 mg de vésicules pour un volume final de 300μl (tampon PBS).

Pour chaque protocole et chaque dose, des groupes de 4 souris étaient traités par :

✓ NP dissoute dans le tampon PBS (I)
✓ NP encapsulée dans les vésicules selon l'invention (II)
✓ NP dissoute dans le tampon PBS, additionné de vésicules vides (III).
✓ NP dans le tampon PBS, émulsionnée à l'adjuvant complet de FREUND. Le mélange est réalisé extemporanément juste avant l'injection, à raison de 1 volume de solution tampon contenant la protéine avec 1 volume d'adjuvant CFA (SIGMA. St Louis USA) (IV)

Les dosages ont été réalisés par ELISPOT sur IgG, 7 jours après la dernière immunisation. Ce dosage consiste à dénombrer les cellules lymphocytes B productrices d'anticorps anti-NP d'isotypes IgG par rapport aux cellules spléniques totales. Le résultat est donc exprimé comme un rapport r de nombre de cellules produisant les anticorps spécifiques sur le nombre total de cellules observées.

1) Protocole 1 immunisation

✓ Très légère amélioration de la réponse par NP encapsulée (r= $500 \times 10^{-7}$) par rapport à NP seule (r= $400 \times 10^{-7}$).
✓ Pas d'effet d'amplification avec les vésicules vides associées à NP non encapsulée (r= $400 \times 10^{-7}$)

2) Protocole 3 immunisations

✓ NP encapsulée très nettement supérieur (r= $900 \times 10^{-7}$) à NP seule (r= $250 \times 10^{-7}$) et comparable à NP avec adjuvant complet de FREUND (r= $900 \times 10^{-7}$)
✓ NP libre associée aux vésicules vides similaire à NP seule (r= $300 \times 10^{-7}$).

Cet exemple confirme complètement, sur un autre modèle, les résultats de l'exemple 3, et indique que les valeurs d'amplification de la réponse obtenue par encapsulation dans les vésicules multilamellaires selon l'invention sont comparables à celles obtenues avec les adjuvants les plus puissants (l'adjuvant de FREUND est particulièrement efficace, mais du fait de sa toxicité, il n'est pas autorisé dans des formulations à visée vaccinale ou thérapeutique).

**Revendications**

1. Vecteur d'antigène, **caractérisé en ce que** qu'il est constitué d'une dispersion de vésicules multilamellaires au sein desquelles ledit antigène se trouve au moins partiellement encapsulé, lesdites vésicules étant constituées, de leur centre jusqu'à leur périphérie, d'une succession de bicouches concentriques comprenant au moins un agent tensioactif et séparées par un milieu liquide.

2. Vecteur selon la revendication 1, **caractérisé en ce que** lesdites vésicules ont un diamètre inférieur à 20 μm et d'au moins 0,1 μm.

3. Vecteur selon la revendication 2, **caractérisé en ce que** lesdits agents tensioactifs constituant lesdites bicouches sont choisis dans le groupe constitué :

- des phospholipides hydrogénés ou non hydrogénés,
- des acides gras en $C_6$ à $C_{18}$, saturés ou mono- ou polyinsaturés, linéaires ou ramifiés, sous forme d'acide ou de sel d'un métal alcalin, alcalino-terreux ou d'une amine,
- des esters, éthoxylés ou non, de ces mêmes acides gras et

  . de saccharose,
  . de sorbitan,
  . de mannitol,
  . de glycérol ou de polyglycérol,
  . de glycol,

- des mono-, di- ou triglycérides ou des mélanges de glycérides de ces mêmes acides gras,
- des alcools gras en $C_6$ à $C_{18}$, saturés ou mono- ou polyinsaturés, linéaires ou ramifiés, éthoxylés ou non,
- des éthers, éthoxylés ou non, de ces mêmes alcools gras et

- de saccharose,
- de sorbitan,
- de mannitol,
- de glycérol ou de polyglycérol,
- de glycol,

    - des huiles végétales polyéthoxylées, hydrogénées ou non hydrogénées,
    - des polymères séquencés de polyoxyéthylène et de polyoxypropylène,
    - de l'hydroxystéarate de polyéthylèneglycol,
    - des alcools à squelette stérol tel que le cholestérol, le sitostérol.

4. Vecteur selon l'une des revendications 1 à 3, **caractérisé en ce que** les bicouches desdites vésicules comprennent au moins deux agents tensioactifs dont l'un présente une balance hydrophile-lipophile (HLB) comprise entre 1 et 6, de préférence comprise entre 1 et 4, et l'autre une balance hydrophile-lipophile (HLB) comprise entre 3 et 15, de préférence comprise entre 5 et 15.

5. Vecteur selon l'une des revendications 1 à 4, **caractérisé en ce que** le rendement d'encapsulation est supérieur à 50 %, de préférence supérieur à 80 %.

6. Vecteur selon l'une des revendications 1 à 5, **caractérisé en ce que** ledit antigène est un antigène protéique ou peptidique.

7. Vecteur selon la revendication 6, **caractérisé en ce que** ledit antigène protéique est constitué d'une protéine naturelle ou recombinante.

8. Composition contenant au moins un antigène contre lequel on souhaite produire des anticorps spécifiques ou une réponse cellulaire spécifique, **caractérisée en ce que** ledit(lesdits) antigène(s) se trouve(nt) au moins partiellement encapsulé(s) dans les vésicules multilamellaires d'un vecteur tel que défini selon l'une des revendications 1 à 7.

9. Composition selon la revendication 8, **caractérisée en ce qu'**il s'agit d'une composition pharmaceutique, en particulier d'un vaccin.

10. Composition selon la revendication 8, **caractérisée en ce qu'**il s'agit d'une composition destinée à la production d'anticorps monoclonaux.

11. Procédé de préparation d'une composition selon l'une des revendications 8 à 10, **caractérisé en ce qu'**il comprend les étapes de :

    - préparation d'une phase lamellaire cristal-liquide comprenant au moins un agent tensioactif et incorporant le (les) antigène(s),
    - transformation de la phase cristal-liquide en vésicules multilamellaires à structure en oignon, par cisaillement,
    - dispersion dans une solution aqueuse pour obtenir la concentration voulue en antigène(s).

12. Procédé selon la revendication 11, **caractérisé en ce que** ledit cisaillement est un cisaillement homogène.

13. Procédé selon l'une des revendications 11 ou 12, **caractérisé en ce qu'**on utilise au moins 2 tensioactifs dont l'un présente une HLB comprise entre 1 et 6, de préférence entre 1 et 4, et l'autre une HLB comprise entre 3 et 15, de préférence entre 5 et 15.

14. Procédé selon l'une des revendications 11 à 13, **caractérisé en ce que** ledit antigène est un antigène protéique ou peptidique.

**Patentansprüche**

1. Antigenvektor, **dadurch gekennzeichnet, daß** er aus einer Dispersion multilamellarer Bläschen besteht, innerhalb derer das Antigen wenigstens teilweise eingekapselt ist, wobei die Bläschen von ihrer Mitte bis zu ihrem Umfang aus einer Folge zweischichtiger konzentrischer Zusammensetzungen bestehen, die wenigstens ein Tensid umfassen und durch ein flüssiges Medium getrennt sind.

2. Vektor nach Anspruch 1, **dadurch gekennzeichnet, daß** die Bläschen einen Durchmesser von weniger als 20 µm und von wenigstens 0,1 µm aufweisen.

3. Vektor nach Anspruch 2, **dadurch gekennzeichnet, daß** die Tenside, aus denen die zweischichtigen Zusammensetzungen bestehen, ausgewählt sind aus der Gruppe, bestehend aus:

- hydrogenierten oder nicht hydrogenierten Phospholipiden,
- gesättigten oder einfach oder mehrfach ungesättigten, linearen oder verzweigten Fettsäuren in $C_6$ bis $C_{18}$ in Form von Säure oder Salz eines Alkali- oder Erdalkalimetalls oder eines Amins,
- ethoxylierten Estern dieser Fettsäuren und
    Saccharose,
    Sorbitan,
    Mannitol,
    Glycerol oder Polyglycerol,
    Glycol,
- Mono-, Di- oder Triglyceriden oder Gemischen aus Glyceriden dieser Fettsäuren
- gesättigten oder einfach oder mehrfach ungesättigten, linearen oder verzweigten, ethoxylierten oder nicht ethoxylierten Fettalkoholen in $C_6$ bis $C_{18}$,
- ethoxylierten oder nicht ethoxylierten Ethern dieser Fettalkohole und
    Saccharose,
    Sorbitan,
    Mannitol,
    Glycerol oder Polyglycerol,
    Glycol,
- polyethoxylierten, hydrogenierten oder nicht hydrogenierten Pflanzenölen,
- sequenzierten Polymeren on Polyoxyethylen und Polyoxypropylen,
- Polyethylenglycol-Hydroxystearat,
- Alkoholen mit Sterolskelett, wie z.B. Cholesterin, Sitosterol.

4. Vektor nach einem der Ansprüche 1 bis 3, **dadurch gekennzeichnet, daß** die zweischichtigen Zusammensetzungen der Bläschen wenigstens zwei Tenside umfassen, von denen eines eine hydrophil lipophile Balance (HLB) aufweist, die zwischen 1 und 6 liegt, vorzugsweise zwischen 1 und 4, und das andere eine hydrophillipophile Balance (HLB) aufweist, die zwischen 3 und 15 liegt, vorzugsweise zwischen 5 und 15.

5. Vektor nach einem der Ansprüche 1 bis 4, **dadurch gekennzeichnet, daß** der Einkapselungsertrag über 50 %, vorzugsweise über 80 % beträgt.

6. Vektor nach einem der Ansprüche 1 bis 5, **dadurch gekennzeichnet, daß** es sich bei dem Antigen um ein Protein- oder Peptidantigen handelt.

7. Vektor nach Anspruch 6, **dadurch gekennzeichnet, daß** das Proteinantigen aus einem natürlichen oder rekombinierenden Protein besteht.

8. Zusammensetzung, die wenigstens ein Antigen enthält, gegen das spezifische Antikörper oder eine spezifische Zellreaktion produziert werden sollen, **dadurch gekennzeichnet, daß** das(die) Antigene wenigstens teilweise in den multilamellaren Bläschen eines Vektors, wie er nach einem der Ansprüche 1 bis 7 definiert ist, eingekapselt ist(sind).

9. Zusammensetzung nach Anspruch 8, **dadurch gekennzeichnet, daß** es sich um eine pharmazeutische Zusammensetzung, insbesondere einen Impfstoff handelt.

10. Zusammensetzung nach Anspruch 8, **dadurch gekennzeichnet, daß** es sich um eine Zusammensetzung handelt, die zur Produktion monoklonaler Antikörper bestimmt ist.

11. Verfahren zur Herstellung einer Zusammensetzung nach einem der Ansprüche 8 bis 10, **dadurch gekennzeich-**

**net, daß** sie folgende Schritte umfaßt:

- Herstellung einer kristallflüssigen lamellaren Phase, die wenigstens ein Tensid umfaßt und das(die) Antigen (e) enthält,
- Umwandlung der kristallflüssigen Phase in multilamellare Bläschen mit Zwiebelstruktur durch Aufschneiden,
- Dispersion in einer wäßrigen Lösung, um die gewünschte

Antigenkonzentration zu erhalten.

12. Verfahren nach Anspruch 11, **dadurch gekennzeichnet, daß** es sich bei dem Aufschneiden um ein homogenes Aufschneiden handelt.

13. Verfahren nach einem der Ansprüche 11 oder 12, **dadurch gekennzeichnet, daß** wenigstens 2 Tenside verwendet werden, von denen eines eine HLB zwischen 1 und 6, vorzugsweise zwischen 1 und 4, und das andere eine HLB zwischen 3 und 15, vorzugsweise zwischen 5 und 15, aufweist.

14. Verfahren nach einem der Ansprüche 11 bis 13, **dadurch gekennzeichnet, daß** es sich bei dem Antigen um ein Protein- oder Peptidantigen handelt.

**Claims**

1. Antigen vector, **characterized in that** it consists of a dispersion of multilamellar vesicles within which said antigen is at least partially encapsulated, said vesicles consisting from their center to their periphery of a stacking of concentric bilayers comprising at least one surfactant and being separated by a liquid medium.

2. Vector according to claim 1, **characterized in that** said vesicles have a diameter which is below 20 $\mu$m and of at least 0.1 $\mu$m.

3. Vector according to claim 2, **characterized in that** said surfactants constituting said bilayers are selected from the group consisting of:

- hydrogenated or non-hydrogenated phospholipids,
- linear or branched, saturated or mono- or polyunsaturated $C_6$ to $C_{18}$ fatty acids in the form of the acid or an alkali metal, alkaline earth metal or amine salt,
- ethoxylated or non-ethoxylated esters of these same fatty acids with

  . sucrose,
  . sorbitan,
  . mannitol,
  . glycerol or polyglycerol, or
  . glycol,

- mono-, di- or triglycerides or mixtures of glycerides of these same fatty acids,
- ethoxylated or non-ethoxylated, linear or branched, saturated or mono- or polyunsaturated $C_6$ to $C_{18}$ fatty alcohols,
- ethoxylated or non-ethoxylated ethers of these same fatty alcohols with

  . sucrose,
  . sorbitan,
  . mannitol,
  . glycerol or polyglycerol, or
  . glycol,

- hydrogenated or non-hydrogenated, polyethoxylated vegetable oils,
- polyoxyethylene and polyoxypropylene block polymers,
- polyethylene glycol hydroxystearate, and
- alcohols with a sterol skeleton, such as cholesterol and sitosterol.

4. Vector according to one of claims 1 to 3, **characterized in that** the bilayers of said vesicles comprise at least two surfactants, one of which has a hydrophilic-lipophilic balance (HLB) of between 1 and 6, preferably of between 1 and 4, while the other has a hydrophilic-lipophilic balance (HLB) of between 3 and 15, preferably of between 5 and 15.

5. Vector according to one of claims 1 to 4, **characterized in that** the encapsulation yield is greater than 50%, preferably greater than 80%.

6. Vector according to one of claims 1 to 5, **characterized in that** said antigen is a protein or peptide antigen.

7. Vector according to claim 6, **characterized in that** said protein antigen consists of a natural or recombinant protein.

8. Composition containing at least one antigen against which it is desired to produce specific antibodies or a specific cellular response, **characterized in that** said antigen(s) is (are) at least partially encapsulated in the multilamellar vesicles of a vector as defined according to one of claims 1 to 7.

9. Composition according to claim 8, **characterized in that** it is a pharmaceutical composition, particularly a vaccine.

10. Composition according to claim 8, **characterized in that** it is a composition for the production of monoclonal antibodies.

11. Process for the preparation of a composition according to one of claims 8 to 10, **characterized in that** it comprises the following steps:

    - preparation of a lamellar liquid-crystal phase comprising at least one surfactant and incorporating the antigen (s),
    - transformation of the liquid-crystal phase into onion-structured multilamellar vesicles by shear, and
    - dispersion in an aqueous solution to give the desired concentration of antigen(s).

12. Process according to claim 11, **characterized in that** said shear is homogeneous shear.

13. Process according to claim 11 or 12, **characterized in that** at least 2 surfactants are used, one of which has an HLB of between 1 and 6, preferably between 1 and 4, while the other has an HLB of between 3 and 15, preferably of between 5 and 15.

14. Process according to one of claims 11 to 13, **characterized in that** said antigen is a protein or peptide antigen.

FIG.1

FIG.2

FIG.3

FIG.4

FIG.5

FIG.6